# EUROPÄISCHE PATENTANMELDUNG

(11) **EP 1 270 684 A2**
(43) Veröffentlichungstag der Anmeldung: **02.01.2003**
(21) Anmeldenummer: 02011548.1
(22) Anmeldetag: 24.05.2002
(51) Int. Cl.: C09C 1/00

(54) **Mehrschichtsysteme mit optischen Eigenschaften**

(30) Priorität: 12.06.2001 DE 10128491
(71) Anmelder: MERCK PATENT GmbH, 64293 Darmstadt (DE)
(72) Erfinder: Andes, Stephanie, 63452 Hanau (DE); Fuchs-Pohl, Gerald, Dr., 64331 Weiterstadt (DE); Friz, Martin, Dr., 64297 Darmstadt (DE); Honeit, Ute, 64285 Darmstadt (DE); Pfaff, Gerhard, Dr., 64839 Münster (DE); Uhlig, Michael, 64297 Darmstadt (DE)

(57) **Zusammenfassung**

Die vorliegende Erfindung betrifft Mehrschichtsysteme mit optischen Eigenschaften auf der Basis metallischer Substrate, welche sowohl eine Schicht (A) aus mindestens zwei dielektrischen Materialien unterschiedlicher Brechzahl umfassen, wobei die Brechzahl an der unteren Seite und die Brechzahl an der oberen Seite der Schicht (A) verschieden sind, als auch eine selektiv oder nicht selektiv absorbierende Schicht (B) aufweisen, ein Verfahren zu deren Herstellung sowie deren Verwendung.

## Beschreibung

Die Erfindung betrifft Mehrschichtsysteme mit optischen Eigenschaften auf der Basis von metallischen Substraten, ein Verfahren zu deren Herstellung sowie deren Verwendung.

Mehrschichtige Systeme mit optischen Eigenschaften, welche zentrale Schichten aus reflektierenden Materialien, vorzugsweise Metallen, enthalten, sind insbesondere in Pigmentform bekannt und finden breite Verwendung in vielen Bereichen der Technik, so zum Beispiel für die Herstellung von Autolacken und dekorativen Beschichtungsmaterialien sowie zum Pigmentieren von Kunststoffen, Farben, Druckfarben, insbesondere für den Sicherheitsdruck, Papier, und dergleichen mehr.

In JP H7-759(A) wird ein mehrschichtiges Interferenzpigment mit metallischem Glanz offenbart, welches aus einem Substrat aus Aluminium-, Gold- oder Silberplättchen oder Plättchen aus Glimmer oder Glas, die mit Metallen beschichtet sind, und darauf befindlichen alternierenden Schichten aus Titandioxid und Siliziumdioxid besteht. Dieses Pigment besitzt ein hohes Deckvermögen. Der metallische Kern reflektiert das auftreffende Licht jedoch sehr stark, so dass der durch die Metalloxidschichten hervorgerufene Interferenzeffekt nur in sehr geringem Maße erkennbar ist und der harte metallische Glanz das Erscheinungsbild der Pigmente dominiert.

In US 4,434,010 sind optische Schichtsysteme mit einer zentralen Schicht aus einem opaken, reflektierenden Material, zum Beispiel Aluminium, Gold, Kupfer oder Silber beschrieben, welche auf beiden Seiten mit einer ersten Schicht aus einem niedrigbrechenden dielektrischen Material wie Siliziumdioxid, Magnesiumfluorid oder Aluminiumoxid und einer zweiten, semiopaken Metallschicht aus Chrom, Nickel oder Inconel beschichtet sind.

Diese Schichtsysteme werden vorrangig für den Druck von Wertpapieren oder die Erzeugung von fälschungssicheren Materialien eingesetzt und zeigen mit dem Betrachtungswinkel wechselnde Farben. Werden sie jedoch als Pigmente eingesetzt, sind sie auf Grund ihres Herstellungsverfahrens nicht auf allen Seiten des Metallkerns vollständig von den äußeren Schichten umhüllt, was zu Verarbeitungsproblemen in Beschichtungslösungen führen kann.

Ein mehrlagiger Interferenzfilm, welcher einen Farbverlauf aufweisen und zur Herstellung von Pigmenten verwendet werden kann, ist in US 6,157,489 beschrieben. Dieser besitzt eine zentrale Reflexionsschicht aus Aluminium, Silber, Kupfer und dergleichen, auf welcher beidseitig Schichten aus hochbrechenden dielektrischen Materialien wie beispielsweise Titandioxid, Zinksulfid oder Yttriumoxid und darauf eine Absorptionsschicht aus Chrom, Nickel, Palladium, Titan etc. aufgebracht sind. Auch diese mehrlagigen Interferenzfilme besitzen, wenn sie pigmentförmig eingesetzt werden sollen, Substrate, die nicht vollständig von den äußeren Schichten umhüllt sind, wodurch wiederum Verarbeitungsprobleme auftreten können.

Aus der DE 44 37 753 sind mehrfach beschichtete metallische Glanzpigmente bekannt, welche auf metallischen Substraten ein Schichtpaket aus
(A) einer farblosen Beschichtung mit einem Brechungsindex n ≤ 1,8 und
(B) einer selektiv absorbierenden Beschichtung mit einem Brechungsindex n ≥ 2,0
sowie gewünschtenfalls zusätzlich
(C) eine äußere, farblose oder selektiv absorbierende, von der darunter liegenden Schicht (B) verschiedene Beschichtung aufweisen.

Dabei besteht die Schicht (A) beispielsweise aus Siliziumdioxid, Aluminiumoxid oder Magnesiumfluorid, während die Schicht (B) aus selektiv absorbierenden hochbrechenden Oxiden oder aus "eingefärbten" farblosen hochbrechenden Oxiden zusammengesetzt ist. Diese Pigmente sollen über interessante koloristische Eigenschaften verfügen und zur Erzeugung eines Farbflops, d.h. eines wechselnden farbigen Erscheinungsbildes in Abhängigkeit vom Betrachtungswinkel, geeignet sein.

Den aus den drei letztgenannten Veröffentlichungen bekannten Pigmenten und Schichtsystemen ist gemeinsam, dass die Interferenzfarbe der Pigmente im Wesentlichen durch die Brechzahl und die Dicke der ersten Schicht auf dem metallischen Substrat, die entweder eine niedrige oder eine hohe Brechzahl aufweist, sowie durch die Farbabsorption der darauf befindlichen Schicht bestimmt wird. Die Winkelabhängigkeit und die Farbintensität der Interferenzfarbe wird dagegen nur durch die Zusammensetzung und Dicke der ersten Schicht gesteuert. Insbesondere ist die Farbintensität und die Anzahl der durchlaufenen Farbtöne sowie die Brillanz der Farben in hohem Maße von der stofflichen Zusammensetzung der ersten Schicht abhängig. Es fehlt daher an Einflussmöglichkeiten, mit denen eine Feineinstellung der Farbbrillanz oder der Intensität der Interferenzfarben im durchlaufenen Farbbereich vorgenommen werden kann.

Aus EP 0 632 821 sind Farbpigmente auf der Basis plättchenförmiger Substrate bekannt, die mit Schichten überzogen sind, welche TiO₂, ein oder mehrere Titansuboxide und mindestens ein Oxid mindestens eines anderen Metalls und/oder Nichtmetalls enthalten, wobei die Konzentration der Titanoxide in der Überzugsschicht in der Nähe der Substratoberfläche groß ist und zur Pigmentoberfläche hin allmählich abnimmt. Das Substrat kann ein mit TiO₂ überzogenes Metallplättchen sein und die Überzugsschicht kann neben TiO₂ und den Titansuboxiden auch SiO₂ enthalten. In diesem Falle wird die vorhandene TiO₂-Schicht auf dem Substrat mit metallischem Si reduziert, um das oder die Titansuboxide zu erzeugen, wobei gleichzeitig SiO₂ entsteht, welches auf der Pigmentoberfläche eine Schutzschicht bildet. Diese Pigmente weisen neben einem guten Deckvermögen auch eine hohe elektrische Leitfähigkeit auf, zeigen jedoch keine Farbänderungen in Abhängigkeit vom Beleuchtungs- oder Betrachtungswinkel.

Es war daher die Aufgabe der Erfindung, Mehrschichtsysteme mit optischen Eigenschaften auf der Basis metallischer Substrate zur Verfügung zu stellen, welche über ein hohes Deckvermögen, eine hohe Farbintensität und/oder Farben mit hoher Brillanz bei gleichzeitiger Winkelabhängigkeit der Interferenzfarbe verfügen und deren gewünschte Farbeigenschaften in einfacher Weise eingestellt werden können, sowie ein Verfahren zu deren Herstellung bereitzustellen und geeignete Verwendungsmöglichkeiten aufzuzeigen.

Diese Aufgabe wird erfindungsgemäß gelöst durch Mehrschichtsysteme mit optischen Eigenschaften, umfassend ein metallisches Substrat und mehrere Schichten, umfassend, in dieser Reihenfolge,
(A) eine Schicht aus mindestens zwei dielektrischen Materialien unterschiedlicher Brechzahl auf dem Substrat mit einer unteren, dem Substrat zugewandten Seite und einer oberen, einer darauffolgenden Schicht zugewandten Seite, wobei die Brechzahl an der unteren Seite und die Brechzahl an der oberen Seite der Schicht verschieden sind, und
(B) eine selektiv oder nicht selektiv absorbierende Schicht.

Optional können die erfindungsgemäßen Mehrschichtsysteme eine zusätzliche äußere Schicht (C) umfassen.

Gegenstand der Erfindung ist ebenfalls ein Verfahren zur Herstellung der oben definierten Mehrschichtsysteme, in welchem metallische Substrate nasschemisch durch Fällung, Hydrolyse und/oder Reduktion von Metallsalzen im wässrigen oder organischen Medium und/oder durch CVDoder PVD -Verfahren mit den Schichten (A), (B) und optional (C) beschichtet werden.

Gegenstand der Erfindung ist zusätzlich auch die Verwendung der oben definierten Mehrschichtsysteme in Farben, Lacken, Druckfarben, Kunststoffen, kosmetischen Formulierungen, keramischen Materialien, Papier, Folien, Verpackungsmaterialien, Gläsern, Pigmentpräparationen, Trockenpräparaten, in Sicherheitsanwendungen und zur Lasermarkierung.

Das metallische Substrat ist stark reflektierend und kann alle für Metalleffekte bekannten Metalle und Legierungen umfassen, so zum Beispiel Eisen, Stahl, insbesondere Edelstahl, Aluminium, Kupfer, Nickel, Chrom, Zink, Zinn, Silber, Gold, Platin, Kobalt, Lanthanide und Titan sowie Mischungen oder Legierungen aus zwei oder mehreren Metallen, wie Messing oder Bronzen.

Substrate gemäß der vorliegenden Erfindung sind metallische Schichten in Form von Filmen oder Partikeln.

Liegt das Substrat als Partikel vor, sind insbesondere alle bekannten handelsüblichen Metallpulver geeignet, die in Wasser weitgehend stabil sind oder durch geeignete Maßnahmen stabilisiert werden können. Diese Metallpulver sind in der Regel plättchenförmig.

Dabei sind plättchenförmige Aluminiumpartikel bevorzugt, welche durch übliche Techniken wie das Herausstanzen aus Folien oder Verdüsungsund Mahlverfahren in einfacher Weise zugänglich sind. Es können auch Aluminiumfolien gebrochen und gemahlen werden, oder es werden grobe Aluminiumpartikel bis auf die gewünschte Größe zerkleinert und anschließend klassiert. Zur Herstellung solcher Partikel sind insbesondere die in US 3,949,139 und WO 00/24946 beschriebenen Verfahren geeignet.

Werden gängige Handelsprodukte aus den oben genannten Metallen eingesetzt, so sollten deren Oberflächen jedoch weitestgehend fettfrei sein, was durch Behandlung mit geeigneten Lösungsmitteln oder durch oxydative Behandlung, zum Beispiel gemäß DE-A-42 23 384, erreicht werden kann.
Es ist auch bevorzugt, wenn die metallischen Substrate vor der Beschichtung einer Passivierungsbehandlung unterzogen werden, wie sie beispielsweise in DE 42 36 332 und DE 44 14 079 beschrieben ist. Dadurch wird der Einsatz der erfindungsgemäßen Mehrschichtsysteme in Pigmentform auch in wässrigen Beschichtungssystemen problemlos möglich.

Die Größe der metallischen Substratteilchen wird an den jeweiligen Verwendungszweck der erfindungsgemäßen pigmentförmigen Mehrschichtsysteme angepasst und ist an sich nicht kritisch. Üblicherweise liegt der mittlere Durchmesser der Substratteilchen im Bereich von etwa 1 bis 250 µm, vorzugsweise 2 bis 200 µm und insbesondere 5 bis 50 µm, während die mittlere Dicke zwischen 0,02 und 3 µm, vorzugsweise zwischen 0,05 und 2 µm beträgt.
Die nach dem BET-Verfahren gemessene spezifische Oberfläche beträgt im allgemeinen 0,5-30 m²/g.
Werden Aluminiumplättchen eingesetzt, so weisen diese in der Regel eine mittlere Dicke von größer als 0,05 bis 1µm, einen mittleren Durchmesser von 2 bis 100 µm sowie eine spezifische BET-Oberfläche von 0,5 bis 30 m²/g auf.

Wenn das Substrat als Film vorliegt, kann es sowohl lichtundurchlässig als auch teilweise lichtdurchlässig sein. Im allgemeinen beträgt die Schichtdicke dieser Substrate 0,005 bis 2 µm.

Das Substrat ist dabei entweder ein- oder beidseitig mit den Schichten (A) und (B) und gegebenenfalls (C) beschichtet. Auf diese Weise sind unsymmetrische oder symmetrische Mehrschichtsysteme mit optischen Eigenschaften erhältlich.

Derartige Systeme lassen sich vorteilhaft unter Verwendung von herkömmlichen PVD-Vakuumbandbeschichtungsverfahren herstellen.

Die Filme liegen prozessbedingt zunächst großflächig vor und können in dieser Form direkt weiterverwendet, oder über geeignete Maßnahmen in die gewünschte Anwendungsform gebracht werden.
Hierzu kommen insbesondere Verfahren wie Mahlen, Stanzen, Schneiden, Prägen und dergleichen in Betracht.

Als Materialien unterschiedlicher Brechzahl für die Schicht (A) eignen sich sowohl die bekannten, als niedrigbrechend bezeichneten Materialien mit Brechzahlen von n ≤ 1,8 als auch die bekannten, als hochbrechend bezeichneten Materialien mit Brechzahlen von n > 1,8. Der Unterschied in der Brechzahl n der eingesetzten Materialien sollte dabei mindestens 0,1, bevorzugt jedoch mindestens 0,3, betragen. Dabei ist nicht festgelegt, welche der beiden Seiten der Schicht (A) die größere Brechzahl aufweist.

Es kann in Abhängigkeit von den gewünschten Farbeffekten bestimmt werden, ob die untere Seite der Schicht (A) eine geringere oder höhere Brechzahl als die obere Seite der Schicht (A) aufweisen soll. Mit steigender Dicke der Schicht (A) erfolgt daher vom Substrat aus gesehen entweder ein Anstieg oder Abfall der Brechzahl, welcher kontinuierlich oder stufenweise verlaufen kann, vorzugsweise jedoch kontinuierlich verläuft. Das wird über einen Aufbau der Schicht (A) erreicht, bei welchem in Abhängigkeit von der Entfernung zur Substratoberfläche unterschiedliche Konzentrationen an Materialien mit geringerer oder höherer Brechzahl vorliegen.

Beispielsweise ist an der unteren, dem Substrat zugewandten Seite der Schicht (A) ausschließlich ein Material mit einer geringeren Brechzahl vorhanden, dessen Konzentration jedoch über die Dicke der Schicht bis zur oberen, der darauffolgenden Schicht zugewandten Seite der Schicht (A) abnimmt, während gleichzeitig die Konzentration eines weiteren Materials mit einer höheren Brechzahl in der Art zunimmt, dass dieses an der oberen Seite der Schicht (A) als alleiniges Material vorliegt. In der gleichen Art ist ein Aufbau der Schicht (A) in umgekehrter Reihenfolge der Brechzahlen möglich, d. h., dass die Konzentration eines Materials mit höherer Brechzahl an der unteren Seite der Schicht hoch ist und über die Schichtdicke zur oberen Seite der Schicht hin abnimmt, während gleichzeitig die Konzentration eines Materials mit niedrigerer Brechzahl ansteigt.

Es ist nicht Voraussetzung, dass mindestens eines der Materialien mit unterschiedlicher Brechzahl an einer Seite der Schicht (A) allein vorhanden ist. Vielmehr kann bereits an der unteren Seite der Schicht (A) eine Mischung aus zwei oder mehreren Materialien mit unterschiedlicher Brechzahl vorliegen, deren Verhältnis zueinander sich über die Dicke der Schicht (A) zur oberen Seite hin verändert, wobei aber an der oberen Seite immer noch ein Gemisch vorliegt. So ist es, bei zwei Materialien unterschiedlicher Brechzahl, zum Beispiel möglich, dass das Verhältnis Material mit niedrigerer Brechzahl zu Material mit höherer Brechzahl an der unteren Seite der Schicht (A) 20:1 und an der oberen Seite der Schicht (A) 1:20 beträgt. Es sind jedoch im allgemeinen alle Mischungsverhältnisse geeignet, bei denen sich ein deutlicher Unterschied in der Brechzahl, welcher mindestens 0,1 und vorzugsweise mindestens 0,3 beträgt, zwischen der unteren Seite und der oberen Seite der Schicht (A) feststellen lässt.

Eine weitere Ausführungsform der vorliegenden Erfindung umfasst an der unteren Seite der Schicht (A) eine Mischung aus zwei oder mehreren Materialien mit unterschiedlicher Brechzahl, deren Verhältnis zueinander sich über die Dicke der Schicht (A) zur oberen Seite hin derart verändert, dass das Material, dessen Konzentration von der unteren Seite zur oberen Seite der Schicht (A) ansteigt, an der oberen Seite als alleiniges Material vorliegt. Ebenso ist der umgekehrte Schichtaufbau möglich, bei dem an der unteren Seite der Schicht (A) ein Material allein vorhanden ist, dessen Konzentration sich über die Dicke der Schicht (A) vermindert, während die Konzentration eines weiteren Materials mit verschiedener Brechzahl in der Art ansteigt, dass an der oberen Seite der Schicht (A) eine Mischung aus beiden Materialien vorliegt.

Überraschenderweise wurde festgestellt, dass die Farbeigenschaften des resultierenden Mehrschichtsystems wesentlich dadurch beeinflusst werden können, ob sich an der unteren, dem Substrat zugewandten Seite der Schicht (A) ein Material oder Materialgemisch mit einer höheren oder ein Material oder Materialgemisch mit einer niedrigeren Brechzahl befindet.

Wird auf dem metallischen Substrat zuerst ein Material oder Materialgemisch mit einer geringeren Brechzahl aufgebracht, dessen Konzentration mit zunehmender Schichtdicke abfällt, wobei gleichzeitig die Konzentration eines Materials oder Materialgemisches mit einer höheren Brechzahl ansteigt, so lassen sich gegenüber dem Aufbringen von Schichten mit homogener Zusammensetzung, bei ansonsten identischem Schichtaufbau, eine Erhöhung der Intensität der Interferenzfarbe bei gleich bleibender Farbbrillanz im Vergleich mit einzelnen Schichten aus Materialien mit Brechzahlen n ≤ 1,8 sowie eine Erhöhung der Intensität der Interferenzfarbe bei verbesserter Farbbrillanz und gleichzeitig verbreitertem Farbbereich, in welchem ein winkelabhängiges Farbspiel zu beobachten ist (es werden mehr Farbtöne durchlaufen), im Vergleich mit einzelnen Schichten aus Materialien mit Brechzahlen n > 1,8 feststellen.

Wird dagegen in umgekehrter Reihenfolge auf dem metallischen Substrat zuerst ein Material oder Materialgemisch mit einer höheren Brechzahl aufgebracht, dessen Konzentration mit zunehmender Schichtdicke abfällt, wobei gleichzeitig die Konzentration eines Materials oder Materialgemisches mit einer niedrigeren Brechzahl ansteigt, so lassen sich gegenüber dem Aufbringen von Schichten mit homogener Zusammensetzung, bei ansonsten identischem Schichtaufbau, eine verbesserte Farbbrillanz bei gleichbleibender Farbintensität im Vergleich mit einzelnen Schichten aus Materialien mit Brechzahlen n ≤ 1,8 sowie eine Erhöhung der Intensität der Interferenzfarbe bei wesentlich verbesserter Farbbrillanz und gleichzeitig verbreitertem Farbbereich, in welchem ein winkelabhängiges Farbspiel zu beobachten ist, im Vergleich mit einzelnen Schichten aus Materialien mit Brechzahlen n > 1,8 feststellen.

Durch die Gestaltung der Reihenfolge des Aufbringens von Materialien mit unterschiedlicher Brechzahlen sowie über die Auswahl der jeweiligen Materialien und die Bestimmung der Dicke der Schicht (A) ergeben sich für den Fachmann daher eine Vielzahl von Möglichkeiten, optisch attraktive Mehrschichtsysteme für die verschiedensten Anwendungsbereiche gezielt herstellen zu können. Die dafür erforderlichen Einzelmaßnahmen sind dem Fachmann allgemein bekannt und erfordern kein erfinderisches Zutun.

Als Material mit einer Brechzahl n ≤ 1,8 eignen sich Metallverbindungen, insbesondere Metalloxide, Metallfluoride, Metalloxidhydrate, Metallphosphate oder deren Gemische, die sich filmartig und dauerhaft aufbringen lassen.
Beispiele hierfür sind SiO₂, SiO(OH)₂, Al₂O₃, AlO(OH), B₂O₃, MgF₂, AlF₃, CeF₃, LaF₃, MgSiO₃ oder Aluminiumphosphat. Es lassen sich jedoch auch organische Monomere oder Polymere, beispielsweise Acrylate, vorzugsweise Methacrylate, und Polytetrafluorethylen einsetzen.

Bevorzugt sind SiO₂, Al₂O₃ und MgF₂ oder deren Gemische und besonders bevorzugt SiO₂ und MgF₂.

Als Material mit einer Brechzahl n > 1,8 werden Metallverbindungen, vorzugsweise Metalloxide, Metallsulfide, Metallnitride oder Metallcarbide oder deren Gemische eingesetzt, beispielsweise TiO₂, ZrO₂, SiO, CeO₂, HfO₂, Pr₂O₃,Y₂O₃, Ta₂O₅, ZnO, SnO₂, Ce₂O₃, Fe₂O₃, Fe₃O₄, BiOCI, ZnS, TiN, Si₃N₄, SiC, bevorzugt jedoch TiO₂, ZrO₂ und Fe₂O₃ und insbesondere TiO₂. Letzteres kann dabei sowohl in einer Rutil- als auch einer Anatasmodifikation vorliegen.

Die Schicht (A) weist eine optische Schichtdicke auf, welche bevorzugt einem ganzzahligen Vielfachen des einfallenden Lichtes der Wellenlänge λ/4 entspricht, wobei als Brechzahl n die über die Schichtdicke gemittelte Brechzahl der Materialien mit niedrigerer und höherer Brechzahl zu Grunde gelegt wird. Die Dicke der Schicht (A) beträgt im allgemeinen 100 bis 1000 nm und insbesondere von 150 bis 600 nm.

Die selektiv oder nichtselektiv absorbierende Schicht (B) ist hinsichtlich der Brechzahl des aufgebrachten Materials oder Materialgemisches nicht beschränkt und kann sowohl hochbrechende wie auch niedrigbrechende Materialien umfassen. Sie ist jedoch mindestens teilweise lichtdurchlässig (semiopak) und muss daher bezüglich ihrer Schichtdicke sorgfältig auf die verschiedenen eingesetzten Materialien abgestimmt werden.

Als Materialien kommen insbesondere Metalle wie beispielsweise Chrom, Wolfram, Kobalt, Nickel, Kupfer, Molybdän, Eisen, Silber, Gold, Palladium, Titan, Vanadium, Niob, Platin, aber auch Aluminium sowie Mischungen oder Legierungen aus zwei oder mehreren Metallen in Betracht.

Ebenso geeignet sind jedoch auch Metalloxide, insbesondere solche, die von sich aus absorbierend sind, aber auch solche, die durch Einlagerung von oder Beschichtungen mit absorbierenden Materialien absorbierend gemacht werden können.

Besonders geeignete Metalloxide sind hierbei die verschiedenen Eisenoxide wie Magnetit, Goethit oder Eisen(III)oxide unterschiedlicher Modifikationen, verschiedene Kobaltoxide (CoO, Co₃O₄), Chrom(III)oxid, Titan(III)oxid und die bekannten farbigen Titansuboxide, verschiedene Vanadiumoxide (VO₂, V₂O₃) oder auch Mischoxide wie Pseudobrookit (Fe₂TiO₅) und Ilmenit (FeTiO₃) sowie deren Mischungen.

Metalloxide, die durch Einlagerung absorbierender Partikel wie Ruß oder Kohlenstoff sowie durch Einlagerung von selektiv absorbierenden Farbmitteln, durch Dotieren mit Metallkationen oder durch Überziehen mit einem ein Farbmittel enthaltenden Film absorbierend gemacht werden können, sind zum Beispiel Zirkondioxid oder Titandioxid, die ebenfalls im Gemisch mit einer oder mehreren der oben genannten Substanzen eingesetzt werden können.

Für die Schicht (B) können jedoch auch Metallsulfide wie Kobaltsulfid, Nickelsulfid, Chromsulfid, Eisensulfid, Wolframsulfid, Molybdänsulfid, Cersulfid sowie deren Gemische untereinander oder mit Metalloxiden oder Metallen eingesetzt werden, sowie auch Metallnitride wie Titannitrid oder Titanoxynitrid.

Die Schichtdicke der Schicht (B) bestimmt sich aus dem eingesetzten Material und dem Erfordernis, dass diese Schicht für das sichtbare Licht mindestens noch teilweise durchlässig sein muss.

Für nichtselektiv absorbierende Materialien liegt die Dicke dieser Schicht bei etwa 5 bis 100 nm, wobei der untere Bereich von 5 bis 25 nm, insbesondere 5 bis 20 nm, für stark absorbierende Metalle wie Chrom und Molybdän ausreichend ist.

Werden dagegen selektiv absorbierende Metalloxide eingesetzt, kann die Dicke der Schicht (B) 5 bis 500 nm, vorzugsweise 10 bis 100 nm, betragen.

In der vorliegenden Erfindung besteht die Schicht (B) vorzugsweise aus Chrom mit einer Schichtdicke von 5 bis 20 nm, aus Fe₂O₃ mit einer Schichtdicke von 10 bis 100 nm, oder aus Aluminium mit einer Schichtdicke von 5 bis 30 nm.

Durch die selektiv oder nicht selektiv absorbierende Schicht (B) wird die Reflexion des auftreffenden sichtbaren Lichtes an dem metallischen Substrat abgeschwächt und der durch die Schicht (A) eingestellte Farbeffekt verstärkt. Insbesondere bei der Einarbeitung der erfindungsgemäßen Mehrschichtsysteme in Form von Pigmenten in die üblichen Farblacksysteme kommen daher die optischen Vorteile dieser Pigmente wie erhöhte Intensität der Interferenzfarben verbunden mit einem hohen Deckvermögen und metallischem Glanz, sowie erweiterte Farbbereiche für den Farbflop und/oder eine hohe Brillanz der Farben gut zur Geltung.

Die erfindungsgemäßen Mehrschichtsysteme können optional auch eine äußere Schutzschicht (C) aufweisen. Diese soll vorzugsweise die darunter liegende Schicht (B) schützen und die Mehrschichtsysteme auf diese Weise stabilisieren. Dies ist insbesondere dann notwendig, wenn die erfindungsgemäßen Mehrschichtsysteme in Pigmentform eingesetzt werden.
Als Materialien für die äußere Schicht (C) können farblose oder selektiv absorbierende Metalloxide wie zum Beispiel SiO₂, SiO(OH)₂, Al₂O₃, AIO(OH), SnO₂, TiO₂, ZrO₂, CeO₂, Fe₂O₃ oder auch Cr₂O₃, die auch phosphat-, chromat-, vanadat- oder phosphathaltig sein können, eingesetzt werden.
Es kann jedoch auch eine Nachbehandlung erfolgen, durch welche sowohl die chemische Stabilität der Mehrschichtsysteme erhöht als auch ihre Handhabung, insbesondere die Erleichterung des Einbringens von pigmentförmigen Mehrschichtsystemen in verschiedene Medien, verbessert werden soll.
Dafür kommen insbesondere Verfahren in Frage, welche in DE 22 15 191, DE 31 51 354, DE 32 35 017, DE 33 34 598, DE 40 30 727, EP 0 649 886, WO 97/29059, WO 99/57204 oder US 5,759,255 beschrieben sind.
Die Schicht (C) ist im allgemeinen etwa 1 bis 500 nm dick.

Die erfindungsgemäßen Mehrschichtsysteme können zwischen dem metallischen Substrat und der Schicht (A) noch eine zusätzliche dielektrische Schicht enthalten, welche aus Metalloxiden, Metallfluoriden, Metallsulfiden, Metallnitriden oder deren Gemischen besteht.

Das Verfahren zur Herstellung der erfindungsgemäßen Glanzpigmente kann sowohl ein Verfahren sein, bei dem auf dem plättchenförmigen metallischen Träger alle Schichten (A), (B) und (C), insbesondere die Schicht (A) und die Schicht (B) nasschemisch durch Fällung, Hydrolyse und/oder Reduktion anorganischer oder organischer Metallverbindungen aufgebracht werden, als auch ein Verfahren, bei dem alle aufzubringenden Schichten (A) und (B) über die Gasphasenzersetzung geeigneter Verbindungen oder ein PVD-Verfahren aufgebracht werden, oder ein Verfahren, bei dem je nach Zusammensetzung der Schichten (A) und (B) mehrere Verfahren in Kombination eingesetzt werden.

Ein nasschemisches Verfahren sowohl für die Schicht (A) als auch für die Schicht (B) kommt nur dann in Frage, wenn neben der Schicht (A) auch die Schicht (B) aus nasschemisch abscheidbaren Materialien, beispielsweise aus selektiv absorbierenden Metalloxiden oder aber auch aus bestimmten Metallen, zusammengesetzt ist und wenn ein partikelförmiges Substrat eingesetzt wird.

Als nasschemisches Verfahren kommt dabei sowohl die Fällung, Hydrolyse und/oder Reduktion metallorganischer wie auch anorganischer Metallverbindungen in Betracht, wobei die zur Herstellung von Perlglanzpigmenten entwickelten Beschichtungsverfahren angewendet werden; derartige Verfahren sind z.B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren Patentdokumenten und sonstigen Publikationen.

Bei der Fällung anorganischer Metallverbindungen auf partikelförmige Substrate werden die Substratpartikel in Wasser suspendiert und mit einem oder mehreren hydrolysierbaren Metallsalzen bei einem für die Hydrolyse geeigneten pH-Wert versetzt, der so gewählt wird, dass die Metalloxide bzw. Metalloxidhydrate direkt auf den Partikeln niedergeschlagen werden, ohne dass es zu Nebenfällungen kommt. Der pH-Wert wird üblicherweise durch gleichzeitiges Zudosieren einer Säure oder Base konstant gehalten. Anschließend werden die Pigmente abgetrennt, gewaschen und getrocknet und gegebenenfalls kalziniert, wobei die Temperatur im Hinblick auf die jeweils vorliegende Beschichtung optimiert werden kann. Falls gewünscht können die Pigmente nach Aufbringen einzelner Beschichtungen abgetrennt, getrocknet und ggf. kalziniert werden, um dann zur Auffällung der weiteren Schichten wieder resuspendiert zu werden.

Die Schicht (A) kann mit Hilfe dieses Verfahrens derart auf das Substrat aufgebracht werden, dass entweder eine stufenförmige oder aber eine kontinuierliche Änderung der Brechzahl innerhalb der Schicht erfolgt. Soll zum Beispiel die Brechzahl vom Substrat aus gesehen über die Schichtdicke hin ansteigen, so wird mit dem Ausfällen eines Materials mit niedrigerer Brechzahl oder mit einem Gemisch, bei dem das Material mit niedrigerer Brechzahl überwiegt, auf dem Substrat begonnen. Mit fortschreitender Reaktion kann entweder ein Material mit höherer Brechzahl laufend zudosiert werden, wobei die Menge des Materials mit niedrigerer Brechzahl stetig verringert wird, oder es werden stufenförmig in kurzen Zeitabständen jeweils neue Mischverhältnisse von Mischungen des Materials mit niedrigerer Brechzahl und des Materials mit höherer Brechzahl zugegeben, wobei sehr dünne Einzelschichten mit jeweils verändertem Mischungsverhältnis der beiden Komponenten entstehen. In der gleichen Weise kann ein umgekehrter Schichtaufbau erfolgen.
In der Praxis hat sich hierzu das in J. Mater. Chem., 2001, 11, 984-986 beschriebene Verfahren als vorteilhaft erwiesen.

Metallorganische Verbindungen, wie zum Beispiel die Metallalkoholate, werden in Gegenwart der Substratteilchen und eines organischen Lösungsmittels, welches mit Wasser mischbar ist und in welchem die Metallverbindungen löslich sind, hydrolysiert. Werden zum Beispiel Tetraethoxysilan oder Aluminiumtriisopropanolat verwendet, können diese in Gegenwart eines Alkohols, insbesondere Isopropanol, und von wässrigem Ammoniak als Katalysator, hydrolytisch zersetzt werden. Auf diese Weise kann das Substrat mit einer SiO₂ oder Al₂O₃-Schicht beschichtet werden. Dieses Verfahren wird in DE 44 05 492 genauer beschrieben.
Nach und nach wird zu dieser Lösung eine organische Verbindung eines Materials mit höherer Brechzahl, beispielsweise Tetrabutylorthotitanat oder Tetraethylorthotitanat zudosiert, während die Zufuhr von Tetraethoxysilan oder Aluminiumtriisopropanolat vermindert wird.
Die Materialien können auch in umgekehrter Reihenfolge aufgebracht werden.

Weiterhin können die einzelnen Schichten der erfindungsgemäßen Pigmente auch in einem Wirbelbettreaktor durch Gasphasenbeschichtung abgeschieden werden, wobei z.B. die in EP 0 045 851 und EP 0 106 235 zur Herstellung von Perlglanzpigmenten vorgeschlagenen Verfahren entsprechend angewendet werden.

Die einzelnen Schichten können auch nach bekannten Verfahren durch Sputtern von Metallen, beispielsweise von Aluminium oder Chrom oder von Legierungen, wie zum Beispiel Chrom-Nickel-Legierungen sowie von Metalloxiden, beispielsweise von Titanoxid, Siliciumoxid oder Indium-Zinn-Oxid oder durch thermisches Verdampfen von Metallen oder Metalloxiden hergestellt werden.

Im folgenden soll das Aufbringen der Schichten durch Aufdampfen näher beschrieben werden:

Für die Herstellung des Schichtsystems auf dem Substrat kann eine Bedampfungsanlage eingesetzt werden, die aus den üblichen Komponenten, wie Vakuumkessel, Vakuumpumpsystem, Druckmess- und Steuereinheiten, Verdampfereinrichtungen, wie Widerstandsverdampfer oder Elektronenstrahlverdampfer, Vorrichtung zur Einstellung bestimmter Druckverhältnisse sowie einem Gaseinlass- und Regelsystem für Sauerstoff besteht.

Die Hochvakuumaufdampftechnik ist ausführlich beschrieben in Vakuum-Beschichtung, Bände 1-5; Herausgeber Frey, Kienel u. Löbl, VDI-Verlag 1995.

Das Aufbringen der Schichten durch Sputter-Verfahren erfolgt auf folgende Weise:

Beim Sputterverfahren oder bei der Kathodenzerstäubung wird zwischen dem Träger und dem Beschichtungsmaterial, das in Form von Platten (Target) vorliegt, eine Gasentladung (Plasma) gezündet. Das Beschichtungsmaterial wird durch energiereiche Ionen aus dem Plasma, z.B. Argonionen, beschossen und dadurch abgetragen bzw. zerstäubt. Die Atome oder Moleküle des zerstäubten Beschichtungsmaterials werden auf dem Substrat niedergeschlagen und bilden die gewünschte dünne Schicht. Zum Aufbringen eines Gemisches aus beispielsweise zwei verschiedenen Beschichtungsmaterialien unterschiedlicher Brechzahl liegen gleichzeitig zwei verschiedene Targets vor, welche durch unterschiedliche Energiezufuhr in unterschiedlichen Konzentrationen auf dem Substrat niedergeschlagen werden.

Dabei wird entweder kontinuierlich die Energiezufuhr gesteigert oder vermindert, oder es werden stufenweise jeweils bestimmte Mischungsverhältnisse abgeschieden, welche durch Neueinstellung von Stoffmengen oder Energiezufuhr in kurzen Abständen verändert werden.

Für Sputterverfahren eignen sich besonders Metalle oder Legierungen. Diese können mit vergleichsweise hohen Geschwindigkeiten, insbesondere im sogenannten DC-Magnetron-Verfahren, zerstäubt werden. Verbindungen wie Oxide oder Suboxide oder Mischungen aus Oxiden können durch Einsatz des Hochfrequenz-Sputtern ebenfalls zerstäubt werden. Die chemische Zusammensetzung der Schichten wird durch die Zusammensetzung des Beschichtungsmaterials (Target) bestimmt. Sie kann aber auch durch Zusätze zum Gas, das das Plasma bildet, beeinflusst werden. Insbesondere werden Oxid- oder Nitridschichten durch Zusatz von Sauerstoff oder Stickstoff im Gasraum hergestellt.

Die Struktur der Schichten kann durch geeignete Maßnahmen, wie Beschuss der aufwachsenden Schichten durch lonen aus dem Plasma, beeinflusst werden.

Das Sputterverfahren ist ebenfalls beschrieben in Vakuum-Beschichtung, Bände 1-5; Herausgeber Frey, Kienel und Löbl, VDI-Verlag 1995.

Bei der Verwendung partikulärer Substrate ist eine Anpassung des Hochvakuumbedampfungsverfahrens an das in Pulverform vorliegende Substrat unbedingt erforderlich. Dazu ist es notwendig, das Substrat während des Bedampfungsverfahrens im Vakuumkessel gleichmäßig in Bewegung zu halten, um eine homogene Beschichtung aller Partikeloberflächen zu gewährleisten.
Dies gelingt zum Beispiel durch den Einsatz von rotierenden Behältern oder die Verwendung von Vibrationsvorrichtungen.

Für die Herstellung von großflächigen Filmen eignen sich vorzugsweise kontinuierlich oder diskontinuierlich ablaufende PVD-Vakuumbandbeschichtungsverfahren, bei denen die einzelnen Schichten des Schichtsystems nacheinander abgeschieden werden. Auf diese Weise können symmetrische oder unsymmetrische Mehrschichtsysteme gemäß der vorliegenden Erfindung hergestellt werden.
Soll ein filmartiges Mehrschichtsystem erzeugt werden, muss ein geeigneter bandförmiger Träger vorliegen. Dieser Träger besteht aus einem flexiblen Material, zum Beispiel einem Polyester, wie Polyethylenterephthalat. Dieser Träger wird in Abhängigkeit von der gewünschten Weiterverwendung des erfindungsgemäßen Mehrschichtsystems vorzugsweise mit einer in einem Lösungsmittel oder thermisch löslichen Ablöseschicht (release layer) beschichtet, wenn das Mehrschichtsystem vom Träger abgelöst und gegebenenfalls in Pigmentform weiterverwendet werden soll. Der Träger kann jedoch auch ohne weitere Vorbeschichtung sofort mit dem erfindungsgemäßen Mehrschichtsystem beschichtet werden, wenn an eine Verwendung gedacht ist, bei der das erfindungsgemäße Mehrschichtsystem in Form von größeren Flächen oder streifenförmig, kreisförmig oder dergleichen verwendet werden soll. Ebenso besteht die Möglichkeit, den Träger sowohl mit einer Ablöseschicht als auch mit einer Haftschicht zu versehen, bevor das erfindungsgemäße Mehrschichtsystem abgeschieden wird. In diesem Falle kann das Mehrschichtsystem als Fläche vom Träger gelöst werden, um anschließend mittels der Haftschicht auf andere Materialien in Flächenform aufgebracht werden zu können.
Es versteht sich von selbst, dass zur Erzeugung eines symmetrischen Mehrschichtsystems der erfindungsgemäßen Art auf einem gegebenenfalls vorbeschichteten bandförmigen Träger zuerst die äußere Schicht des Systems, d. h. optional die Schicht (C) und anschließend daran die Schichten (B), (A), das metallische Substrat, die Schichten (A), (B) und optional die Schicht (C) aufgebracht werden.
Zur Erzeugung eines unsymmetrischen Mehrschichtsystems wird der Träger gegebenenfalls mit den oben genannten Funktionsschichten vorbeschichtet und anschließend die Schichten (A), (B) und optional (C) in der Art aufgebracht, dass die Schichtenfolge auf beiden Seiten des Substrates verschieden ist.

Liegen die erfindungsgemäßen Mehrschichtsysteme als Pigmente vor, sind sie mit einer Vielzahl von Farbsystemen kompatibel, vorzugsweise aus dem Bereich der Lacke, Farben und Druckfarben. Weiterhin sind diese Pigmente auch in Kunststoffen, keramischen Materialien, Papier, Gläsern, für die Lasermarkierung von Papier und Kunststoffen, in Sicherheitsanwendungen, Folien und Verpackungsmaterialien, sowie für Anwendungen im Agrarbereich, z.B. für Gewächshausfolien, geeignet.
Auf Grund ihrer hohen Farbkraft sind sie insbesondere auch in kosmetischen Formulierungen, zum Beispiel in der dekorativen Kosmetik, vorteilhaft einsetzbar. Sie eignen sich ebenso zur Herstellung von Pigmentpräparationen und Trockenpräparaten, wie zum Beispiel Granulaten, Chips, Pellets, Briketts, etc., welche insbesondere in Druckfarben und Lacken Verwendung finden.

Für die verschiedenen Anwendungszwecke sind die Mehrschichtsysteme in Pigmentform auch vorteilhaft in Abmischung mit handelsüblichen Farbstoffen und Pigmenten, beispielsweise organischen Farbstoffen, organischen Pigmenten oder anderen Pigmenten, wie z.B. transparenten und deckenden Weiß, Bunt- und Schwarzpigmenten sowie mit plättchenförmigen Eisenoxiden, organischen Pigmenten, holographischen Pigmenten, LCPs (Liquid Crystal Polymers), und herkömmlichen transparenten, bunten und schwarzen Glanzpigmenten auf der Basis von metalloxidbeschichteten Glimmer- und SiO₂-Plättchen, etc., einsetzbar.
Die Mehrschichtpigmente können in jedem Verhältnis mit handelsüblichen Pigmenten, Bindemitteln und Füllstoffen gemischt werden.

Werden die erfindungsgemäßen Mehrschichtsysteme in flächiger Form eingesetzt, sind sie insbesondere für die Herstellung von, oder direkt als Folien und Verpackungsmaterialien geeignet. Darunter sollen insbesondere Kaltprägefolien, Heissprägefolien, Laminierfolien, dekorative Folien, Beschichtungsfolien, Schrumpffolien oder Teile derselben verstanden werden.
Eine besondere Bedeutung kommt auch der Anwendung in Sicherheitsanwendungen zu, zum Beispiel als Sicherheitsfäden oder -streifen für Geldscheine, Wertpapiere, Ausweise, Geldkarten, Ausweishüllen oder dergleichen.

Die erfindungsgemäßen Mehrschichtsysteme weisen ein hohes Deckvermögen auf und zeigen intensive Interferenzfarben. Abhängig von der Reihenfolge der aufgebrachten Materialien mit höherer oder niedrigerer Brechzahl sind Farbeffekte wie z.B. eine Verbreiterung des Farbbereiches, in dem abhängig vom Belichtungs- oder Betrachtungswinkel Farbänderungen beobachtet werden können, oder aber eine besondere Farbbrillanz mit weichen Farbübergängen gezielt einstellbar. Diese Vorteile kommen zum Beispiel in den üblichen Farblacksystemen, welche gängige Bindemittel und Zuschlagstoffe enthalten, besonders gut zum Tragen.

Mittels einfacher Beschichtungstechnologien können daher attraktive Mehrschichtsysteme mit optischen Eigenschaften zur Verfügung gestellt werden, welche in vielen Anwendungsbereichen vorteilhaft eingesetzt werden können.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung näher erläutern, ohne sie jedoch zu beschränken.

### Beispiele

### Beispiel 1

Zunächst werden getrennt voneinander die Lösungen A und B angesetzt. Dazu werden 0,5 mol/l (NH₄)₂TiF₆ in voll entsalztem Wasser gelöst für Lösung A, bzw. 0,5 mol/l (NH₄)₂SiF₆ in voll entsalztem Wasser für Lösung B. Beide Lösungen enthalten jeweils zusätzlich 1 mol/l H₃BO₃.
75 g passivierte Aluminium Flakes mit einer Teilchengröße von 10-50 µm und einer mittleren Schichtdicke von 300 nm werden in 1,75 I Lösung A suspendiert , auf 30°C gebracht und bei dieser Temperatur 5 h gerührt (Vorbehandlungszeit).
Anschließend wird über eine Zeitspanne von 20 h mit konstanter Dosierrate 2,33 l Lösung B zudosiert. Simultan dazu wird die entsprechende Menge Mischlösung kontrolliert entnommen, um die Reaktionslösung gleichbleibend bei einem Volumen von ca. 2 l zu halten.
Nach beendeter Reaktionszeit lässt man auf Raumtemperatur abkühlen, filtriert das erhaltene Pigment ab, wäscht mit voll entsalztem Wasser salzfrei und trocknet bei 110°C.
Danach wird das Pulver 30 min bei 500°C kalziniert.

Über ein PVD-Verfahren wird anschließend eine 5 nm dicke Cr-Schicht abgeschieden.

Das fertige Pigment zeigt einen farbintensiven Goldton mit einem besonders fließenden Farbübergang über Grün ins Blau.

### Beispiel 2

Zunächst werden getrennt voneinander die Lösungen A und B angesetzt. Dazu werden 0,5 mol/l (NH₄)₂SiF₆ in voll entsalztem Wasser gelöst für Lösung A, bzw. 0,5 mol/l (NH₄)₂TiF₆ in voll entsalztem Wasser für Lösung B. Beide Lösungen enthalten jeweils zusätzlich 1 mol/l H₃BO₃.
75 g passivierte Aluminium Flakes mit einer Teilchengröße von 10-50µm und einer mittleren Schichtdicke von 300 nm werden in 2,33 l Lösung A suspendiert, auf 30°C gebracht und bei dieser Temperatur 3 h gerührt (Vorbehandlungszeit).
Anschließend wird über eine Zeitspanne von 10 h mit konstanter Dosierrate 1,75 l Lösung B zudosiert. Simultan dazu wird die entsprechende Menge Mischlösung kontrolliert entnommen, um die Reaktionslösung gleichbleibend bei einem Volumen von ca. 2 l zu halten.

Nach beendeter Reaktionszeit lässt man auf Raumtemperatur abkühlen, filtriert das erhaltene Pigment ab, wäscht mit voll entsalztem Wasser salzfrei und trocknet bei 110°C.
Danach wird das Pulver 30 min bei 500°C kalziniert.

Über ein PVD-Verfahren wird anschließend eine 5 nm dicke Cr-Schicht abgeschieden.

Das fertige Pigment zeigt einen farbintensiven Goldton mit einem besonders fließenden Farbübergang ins Grünblau.

## Patentansprüche

1. Mehrschichtsysteme mit optischen Eigenschaften, umfassend ein metallisches Substrat und mehrere Schichten, umfassend, in dieser Reihenfolge,
(A) eine Schicht aus mindestens zwei dielektrischen Materialien unterschiedlicher Brechzahl auf dem Substrat mit einer unteren, dem Substrat zugewandten Seite und einer oberen, einer darauffolgenden Schicht zugewandten Seite, wobei die Brechzahl an der unteren Seite und die Brechzahl an der oberen Seite der Schicht verschieden sind,
und
(B) eine selektiv oder nicht selektiv absorbierende Schicht.

2. Mehrschichtsysteme gemäß Anspruch 1, wobei das metallische Substrat plättchenförmig ist.

3. Mehrschichtsysteme gemäß Anspruch 1, wobei das metallische Substrat in Form eines Filmes vorliegt.

4. Mehrschichtsysteme gemäß einem oder mehreren der Ansprüche 1 bis 3, die zusätzlich eine äußere Schicht (C) aufweisen.

5. Mehrschichtsysteme gemäß einem oder mehreren der Ansprüche 1 bis 4, wobei die Brechzahl von der unteren Seite zu der oberen Seite der Schicht (A) ansteigt.

6. Mehrschichtsysteme gemäß einem oder mehreren der Ansprüche 1 bis 4, wobei die Brechzahl von der unteren Seite zu der oberen Seite der Schicht (A) abfällt.

7. Mehrschichtsysteme gemäß einem der Ansprüche 5 oder 6, wobei die Brechzahl kontinuierlich ansteigt oder abfällt.

8. Mehrschichtsysteme gemäß einem der Ansprüche 5 oder 6, wobei die Brechzahl stufenförmig ansteigt oder abfällt.

9. Mehrschichtsysteme gemäß einem oder mehreren der Ansprüche 1 bis 8, wobei die Brechzahlen von je zwei dielektrischen Materialien in der Schicht (A) eine Differenz von mindestens 0,1 aufweisen.

10. Mehrschichtsysteme gemäß Anspruch 9, wobei die Brechzahlen von je zwei dielektrischen Materialien in der Schicht (A) eine Differenz von mindestens 0,3 aufweisen.

11. Mehrschichtsysteme gemäß einem oder mehreren der Ansprüche 1 bis 10, wobei die Brechzahl an der unteren Seite der Schicht (A) und die Brechzahl an der oberen Seite der Schicht (A) eine Differenz von mindestens 0,1 aufweisen.

12. Mehrschichtsysteme gemäß Anspruch 11, wobei die Brechzahl an der unteren Seite der Schicht (A) und die Brechzahl an der oberen Seite der Schicht (A) eine Differenz von mindestens 0,3 aufweisen.

13. Mehrschichtsysteme gemäß einem oder mehreren der Ansprüche 1 bis 12, wobei das metallische Substrat aus Metallen, Metalllegierungen oder deren Gemischen besteht.

14. Mehrschichtsysteme gemäß einem oder mehreren der Ansprüche 1 bis 13, wobei die Schicht (A) aus Materialien mit einer Brechzahl n ≤ 1,8 besteht.

15. Mehrschichtsysteme gemäß einem oder mehreren der Ansprüche 1 bis 13, wobei die Schicht (A) aus Materialien mit einer Brechzahl n > 1,8 besteht.

16. Mehrschichtsysteme gemäß einem oder mehreren der Ansprüche 1 bis 13, wobei die Schicht (A) aus Materialien mit einer Brechzahl n ≤ 1,8 und Materialien mit einer Brechzahl n > 1,8 besteht.

17. Mehrschichtsysteme gemäß einem der Ansprüche 14 oder 16, wobei das Material mit einer Brechzahl n ≤ 1,8 ein Metalloxid, Metallfluorid, Metalloxidhydrat, Metallphosphat oder ein Gemisch derselben, oder ein organisches Monomer oder Polymer ist.

18. Mehrschichtsysteme gemäß einem der Ansprüche 15 oder 16, wobei das Material mit einer Brechzahl n > 1,8 ein Metalloxid, Metallsulfid, Metallnitrid, Metallcarbid oder ein Gemisch derselben ist.

19. Mehrschichtsysteme gemäß einem oder mehreren der Ansprüche 1 bis 18, wobei die selektiv oder nicht selektiv absorbierende Schicht aus einem mindestens teilweise lichtdurchlässigen Metall oder einem selektiv absorbierenden Metalloxid, Metallsulfid, Metallnitrid oder aus Legierungen oder Gemischen derselben besteht.

20. Mehrschichtsysteme gemäß Anspruch 13, wobei das metallische Substrat aus Eisen, Stahl, Edelstahl, Aluminium, Kupfer, Nickel, Chrom, Zink, Zinn, Silber, Gold, Platin, Kobalt, Lanthaniden, Titan oder Mischungen oder Legierungen derselben besteht.

21. Mehrschichtsysteme gemäß Anspruch 17, wobei das Material mit einer Brechzahl n ≤ 1,8 SiO₂, SiO(OH)₂, Al₂O₃, AIO(OH), B₂O₃, MgF₂, AlF₃, CeF₃, LaF₃, MgSiO₃, Aluminiumphosphat oder ein Gemisch derselben, oder ein Acrylat, Methacrylat oder Polytetrafluorethylen ist.

22. Mehrschichtsysteme gemäß Anspruch 21, wobei das Material mit einer Brechzahl n ≤ 1,8 SiO₂, Al₂O₃, MgF₂, oder ein Gemisch derselben ist.

23. Mehrschichtsysteme gemäß Anspruch 22, wobei das Material mit einer Brechzahl n ≤ 1,8 SiO₂ oder MgF₂ ist.

24. Mehrschichtige Glanzpigmente gemäß Anspruch 18, wobei das Material mit einer Brechzahl n > 1,8 TiO₂, ZrO₂, SiO, CeO₂, HfO₂,Pr₂O₃, Y₂O₃, Ta₂O₅, ZnO, SnO₂, Ce₂O₃, Fe₂O₃, Fe₃O₄, BiOCl, ZnS, TiN, Si₃N₄, SiC, oder ein Gemisch derselben ist.

25. Mehrschichtsysteme gemäß Anspruch 24, wobei das Material mit einer Brechzahl n > 1,8 TiO₂, ZrO₂, Fe₂O₃, oder ein Gemisch derselben ist.

26. Mehrschichtsysteme gemäß Anspruch 25, wobei das Material mit einer Brechzahl n > 1,8 TiO₂ ist.

27. Mehrschichtsysteme gemäß Anspruch 19, wobei die selektiv oder nicht selektiv absorbierende Schicht (B) aus Chrom, Wolfram, Kobalt, Nickel, Kupfer, Molybdän, Aluminium oder Magnetit, Goethit, Eisen(III)oxid, Kobaltoxid, Chrom(III)oxid, Titan(III)oxid, Titansuboxid, Vanadiumoxid, Pseudobrookit, Ilmenit oder Kobaltsulfid, Nickelsulfid, Chromsulfid, Eisensulfid, Wolframsulfid, Molybdänsulfid, Cersulfid oder Titannitrid oder Titanoxynitrid oder aus Gemischen derselben oder aus Legierungen aus zwei oder mehreren Metallen besteht.

28. Mehrschichtsysteme gemäß Anspruch 27, wobei die selektiv oder nicht selektiv absorbierende Schicht (B) aus Chrom, Aluminium oder Eisen(III)oxid besteht.

29. Mehrschichtsysteme gemäß einem oder mehreren der Ansprüche 1 bis 28, welche in Form von Pigmenten vorliegen.

30. Mehrschichtsysteme gemäß einem oder mehreren der Ansprüche 1 bis 28, welche in Form von Filmen vorliegen.

31. Verfahren zur Herstellung der Mehrschichtsysteme gemäß einem oder mehreren der Ansprüche 1 bis 30, wobei ein metallisches Substrat nasschemisch durch Fällung, Hydrolyse oder Reduktion von Metallsalzen im wässrigen oder organischen Medium und/oder durch CVD- oder PVD-Verfahren mit den Schichten (A), (B) und optional(C) beschichtet wird.

32. Verfahren zur Herstellung der Mehrschichtsysteme gemäß Anspruch 31, wobei ein flexibler Träger mit dem metallischen Substrat und den Schichten (A), (B) und optional (C) derart beschichtet wird, dass die Schichten (A), (B) und optional (C) symmetrisch auf beide Seiten des Substrates oder unsymmetrisch nur auf eine Seite des Substrates mit einem PVD- Vakuumbandbeschichtungsverfahren aufgebracht werden.

33. Verwendung der Mehrschichtsysteme gemäß einem oder mehreren der Ansprüche 1 bis 32 in Farben, Lacken, Druckfarben, Kunststoffen, kosmetischen Formulierungen, keramischen Materialien, Papier, Folien, Verpackungsmaterialien, Gläsern, Pigmentpräparationen, Trockenpräparaten, in Sicherheitsanwendungen sowie zur Lasermarkierung.

34. Farben, Lacke, Druckfarben, Kunststoffe, kosmetische Formulierungen, keramische Materialien, Papier, Folien, Verpackungsmaterialien, Gläser, Pigmentpräparationen, Trockenpräparate und Materialien für Sicherheitsanwendungen, enthaltend ein Mehrschichtsystem gemäß einem oder mehreren der Ansprüche 1 bis 32.
